(19) **Europäisches Patentamt / European Patent Office / Office européen des brevets**

(11) **EP 1 952 794 B1**

(12) **EUROPEAN PATENT SPECIFICATION**

(45) Date of publication and mention
of the grant of the patent:
**06.11.2013 Bulletin 2013/45**

(51) Int Cl.:
*A61K 8/06* *(2006.01)*    *A61K 8/31* *(2006.01)*
*A61K 8/34* *(2006.01)*    *A61Q 1/14* *(2006.01)*
*A61Q 19/10* *(2006.01)*

(21) Application number: **08001514.2**

(22) Date of filing: **28.01.2008**

(54) **Cleansing composition**

Reinigungsmittelzusammensetzung

Composition de nettoyage

(84) Designated Contracting States:
**DE FR GB**

(30) Priority: **29.01.2007 JP 2007018540**
**29.01.2007 JP 2007018541**

(43) Date of publication of application:
**06.08.2008 Bulletin 2008/32**

(73) Proprietor: **KAO CORPORATION**
**Chuo-ku**
**Tokyo 103-8210 (JP)**

(72) Inventors:
• **Murase, Yasuyuki**
**Tokyo 131-8501 (JP)**
• **Tsuda, Hiroko**
**Tokyo 131-8501 (JP)**

(74) Representative: **HOFFMANN EITLE**
**Patent- und Rechtsanwälte**
**Arabellastrasse 4**
**81925 München (DE)**

(56) References cited:
EP-A- 1 053 740       JP-A- 4 005 212
JP-A- 4 005 213       JP-A- 4 091 018
JP-A- 6 016 523       JP-A- 6 016 524
JP-A- 2002 114 623    JP-A- 2004 217 640
JP-A- 2004 359 568    JP-A- 2006 117 643

Remarks:
The file contains technical information submitted after the application was filed and not included in this specification

## Description

FIELD OF THE INVENTION

The present invention relates to a transparent liquid cleansing composition.

BACKGROUND OF THE INVENTION

Conventional formulations used for the purpose of dissolving a makeup cosmetic with oil for removal include an emulsifying-type formulation such as a cream or an emulsion in which oil is emulsified in water with a nonionic surfactant, an oil gel-type formulation in which oil is retained in a liquid crystal that is a surfactant structure, or the like, and an oil-type formulation in which a nonionic surfactant is added as an emulsifier to dissolve it in oil.
A durable makeup cosmetic such as a waterproof-type mascara has recently come into fashion, and the waterproof-type mascara forms a wax-adhered coating, and therefore the coating cannot be removed unless dissolved by oil. In the emulsifying-type formulation as described above, oil is finely stably dispersed in an aqueous phase, and therefore the intrinsic cleaning power of the oil cannot be exerted and the makeup removing power is not sufficient. There is a problem that although the oil gel-type formulation in which oil is dispersed or solubilized in a surfactant structure has makeup removing power, the formulation is thick in a gel form and thus it must be to repeatedly rubbed in order to sufficiently exert the intrinsic makeup removing power of the oil. Although the oil-type formulation has the highest cleaning power because oil directly acts on dirt, there is a problem that the formulation is oily during use and rinsing and an oily feeling remains.
JP-A-4-5213 describes a one-phase type cleansing composition containing two nonionic surfactants having different HLB values, a water-soluble compound having a hydroxyl group, liquid oil and water and having improved spreadability. However, there is a problem that such a cleansing composition is difficult to maintain stability. In addition, this cleansing composition forms a transparent gel (gel: liquid crystal structure) in some cases, and thus problems of feeling to the touch and thickening are caused. The cleansing composition has a high compounding ratio of oil and the composition itself has an oily feeling, and thus an oily feeling remains also on the skin after wash-off.
JP-A-2000-256124 and JP-A-2000-256132 describe cosmetics for makeup removal prepared using a liquid crystal composition (liquid crystal phase and/or isotropic surfactant continuous phase) containing a nonionic surfactant, a water-soluble substance having a hydroxyl group, polar oil and/or silicone oil and water. Although it is thought that the cosmetics are well compatible with makeup and have a high makeup removing effect, they are not sufficiently satisfiable in terms of removal of durable makeup such as a waterproof-type mascara. Further, with these cosmetics for makeup removal, a transparent gel (gel: liquid crystal structure) may be formed in some cases, and they are thickened into a gel form, so there is thus a problem that they must be rubbed many times in order to sufficiently exert the intrinsic makeup removing power of the oil.
JP-A-2004-339212 describes a cleansing composition for removing durable makeup such as a waterproof-type mascara by using oil having a low viscosity and a specific water-soluble compound. Since this cleansing composition contains a little amount of a surfactant, however, it is difficult to be washed off without an oily feeling.
As described above, oil-type makeup removers and oil gel-type makeup removers forming a liquid crystal structure have disadvantages, and among conventionally known cleansing compositions, there has been no cleansing composition satisfying both requirements that sufficient cleaning power is exhibited for durable makeup such as a waterproof-type mascara and that the composition can be washed off without an oily feeling.
JP 2004 217640 describes a skin detergent composition comprising (A) 3-80wt.% of an oil component, (B) 1-45wt.% of a hydrophilic non-ionic surfactant, (C) 1-45wt.% of a lipophilic amphiphile, (D) 3-80wt.% of a water-soluble solvent and (E) 3-80wt.% of water and having an isotropic liquid phase exhibiting a bicontinuous structure.
EP 1 053 740 describes a liquid crystal composition and cosmetic preparation which may also be used as a make-up remover comprising 10-60% of a non-ionic surfactant, 1-50% of a water-soluble substance having a hydroxyl group, 1-70% of a silicone oil and 10-60% water. The composition is said to have a liquid crystal phase and/or an isotropic surfactant continuous phase.
JP 04091018 discloses a transparent skin cleanser comprising a hydrophilic nonionic (high HLBs) surfactant, a hydroxyl group-containing water-soluble compound such as propylene glycol, a liquid oil such as liquid paraffin or polyisobutene and water.
JP 2006-117643 discloses a microemulsion comprising a hydrophilic nonionic surfactant, water-soluble organic solvents containing hydroxyl groups, an oily component and water.
JP 2004-359568 discloses a transparent bathing agent, comprising (A) 3 to 15wt. % nonionic surfactant having an HLB of less than 14, (B) 2 to 14 wt. % nonionic surfactants having HLB of more than 14, (C) 10 to 40 wt. % dihydric or more alcohol, (D) 1 to 10 wt. % water and an oil, wherein the average HLB of (A) and (B) is 10.5 to 14.5. The dihydric or more alcohol is e.g. glycerol or butylene or propylene glycol.

JP 2002-114623 discloses a clear composition comprising high amounts of oil, nonionic surfactants having HLB 2 to 10, polyhydric alcohol and water.

JP 06-016524 and JP 06-016523 disclose a skin cleanser comprising hydrophilic nonionic surfactants, water-soluble solvents such as glycerol or ethanol, a liquid oil such as paraffin or olive oil, and water. Specific gel compositions are described which include (A) 13.0 wt. % of nonionic surfactants with HLB of 13 and 14, respectively, (B) 12.5 wt. % of liquid paraffin, (C) 28.0 wt. % of sorbitol and 5.0 wt. % of glycerol and 1.0 wt. % of ethanol.

SUMMARY OF THE INVENTION

The present invention provides a transparent liquid cleansing composition containing the following components (A), (B), (C) and (D) :

(A) 10 to 35% by weight of a nonionic surfactant having an HLB value of 8 or more,
(B) 10 to 40% by weight of oil having a viscosity of 15 mPa·s or less at 30°C,
(C) 20 to 60% by weight of a water-soluble solvent, and
(D) 5 to 50% by weight of water,

wherein the water-soluble solvent of the component (C) comprises (C1) a water-soluble solvent having three or more hydroxyl groups in a molecule and (C2) a water-soluble solvent having one or two hydroxyl groups in a molecule and the weight ratio of the component (C1) to the component (C2) is 1:3 to 3:1 and the total content of the components (C) and (D) is 40 to 70% by weight of the cleansing composition.

DETAILED DESCRIPTION OF THE INVENTION

The present invention relates to a cleansing composition that gives substantially no oily feeling during use, can raise waterproof mascara rapidly and is washed off without an oily feeling.

The present inventors have found that a cleansing composition that provides substantially no oily feeling during use, can raise durable mascara or the like rapidly and is washed off without an oily feeling can be obtained by using a specific nonionic surfactant, an oil having a low viscosity, a water-soluble solvent and water at a specific ratio in combination.

The cleansing composition of the present invention is a cleansing composition that provides substantially no oily feeling during use, can raise durable waterproof mascara or the like rapidly and is washed off without an oily feeling. Further, the cleansing composition has excellent stability in a wide temperature range.

The present inventors thought that if oil is solubilized in a formulation mainly containing an aqueous component to provide a solubilized state in which an oil phase also becomes a continuous phase rather than a simply solubilized state, a makeup remover capable of being cleanly washed off without providing an oily feeling during use and rinsing can be produced while taking advantage of an intrinsic makeup raising speed of oil.

In this case, "a solubilized state so that an oil phase becomes a continuous phase" can be confirmed by a state where a makeup raising speed obtained when makeup  (waterproof mascara) is removed by solubilized oil itself equals to a makeup raising speed obtained when the oil is solubilized.

The nonionic surfactant of the component (A) used in the present invention has an HLB value of 8 or more. When it has an HLB value less than 8, it is difficult to be cleanly washed off without an oily feeling, which is not preferable. In this case, HLB (Hydrophile-Lipophile Balance) represents the molecular weight of a hydrophilic group portion occupied in the total molecular weight of a surfactant, and an HLB value for a polyoxyethylene-based nonionic surfactant is determined by the following Griffin's equation.

$$HLB\ value\ =\ E/5$$

E: weight % of a polyoxyethylene portion included in a surfactant molecule

Specific examples of the nonionic surfactant include polyethylene glycol-based surfactants such as polyethylene glycol fatty acid esters such as polyethylene glycol (12) monolaurate, polyethylene glycol alkyl ethers such as polyethylene glycol (20) octyldodecyl ether, polyethylene glycol alkyl phenyl ethers such as polyethylene glycol (20) nonylphenyl ether, polyethylene glycol castor oil derivatives such as polyethylene glycol (50) castor oil, polyethylene glycol hardened castor oil derivatives such as polyethylene glycol (60) hardened castor oil monoisolaurate, polyethylene glycol sorbitan fatty acid esters such as polyethylene glycol (20) sorbitan monostearate, or the like; polyglycerol fatty acid esters such as diglycerol monoisostearate; polyglycerol alkyl ethers such as diglycerol 2-ethylhexyl ether; saccharose fatty acid esters such as saccharose stearate; and alkyl polyglucosides. Further, among these, a nonionic surfactant having a hydrophobic group having 8 or more carbon atoms is preferable due to good rinsing properties.

Further, among these, a polyethylene glycol fatty acid ester, a polyethylene glycol alkyl ether, a polyglycerol fatty acid ester, polyethylene glycol sorbitan fatty acid ester, a saccharose fatty acid ester and an alkyl polyglucoside are preferable due to better cleaning performance for oily dirt and water-soluble dirt. Furthermore, among these, a polyglycerol fatty acid ester and an alkyl polyglucoside are preferable due to good cleaning performance for oily dirt.

One or more components (A) can be used and are contained in an amount of preferably 5 to 50% by weight, more preferably 10 to 30% by weight in the total composition. If the amount of the component (A) is in this range, a resulting cleansing composition is well compatible with makeup, can raise dirt, is not sticky, and can be cleanly washed off without an oily feeling.

The oil of the component (B) used in the present invention is liquid at normal temperature and has a viscosity of 15 mPa·s or less, preferably 10 mPa·s or less at 30°C. Here, the viscosity is measured by BM-type viscometer (manufactured by Tokimec Inc., Measurement condition: Rotor No. 1, 60 rpm).

Such oil having a low viscosity has a high permeability to a fine portion and a high solubility of dirt and thus has high cleaning power, so that it has an excellent cleaning power for oily makeup dirt such as oily mascara. Further, a strong oily feeling is not accompanied and feeling upon use is good.

As such oil, a liquid oil typically used for cosmetics can be used, and there can be used, for example, hydrocarbon oil such as liquid paraffin, liquid isoparaffin, hydrogenated polyisobutene and squalane; ester oil such as cholesteryl isostearate, isopropyl palmitate, isopropyl myristate, neopentyl glycol dicaprate, isopropyl isostearate, octadecyl myristate, cetyl 2-ethylhexanoate, isononyl isononanoate, isotridecyl isononanoate, glycerol tri(2-ethylhexanoate), glycerol tri(caprylate/caprate); ether oil such as alkyl-1,3-dimethylbutyl ether, nonylphenyl ether; methylcyclopolysiloxane such as decamethylcyclopentasiloxane and octamethylcyclotetrasiloxane, silicone oil such as methylpolysiloxane and methylphenylpolysiloxane; animal and plant oils such as olive oil; and terpene oil.

Among these, oil having a molecular weight of 300 or less and further having a branched chain or a cyclic silicone has high cleaning power and is thus more preferable. Specific examples of the oil include isoparaffin such as light liquid isoparaffin, heavy liquid isoparaffin, and hydrogenated polyisobutene, isopropyl myristate, isopropyl palmitate, isononyl isononanoate, octamethyltrisiloxane, octamethylcyclotetrasiloxane and decamethylcyclopentasiloxane. Further, isoparaffin is preferable, and isododecane is more preferable.

Further, branched hydrocarbon oil is preferably contained in the oil of the component (B), and isoparaffin and further isododecane are preferably contained in an amount of 30 to 100% by weight because cleaning power is increased.

Light liquid isoparaffin is generally a mixture of a branched hydrocarbon having 8 to 18 carbon atoms and if a hydrocarbon having a low molecular weight is contained, the isoparaffin has a peculiar smell. Since such a smell is not favored by a user in some cases, preferably, a component of a low molecular weight having 8 to 9 carbon atoms is not contained as much as possible. On the other hand, if the content of a hydrocarbon having 16 to 18 carbon atoms is higher, its cleaning power is poor and an oily feeling left after use tends to be strong. From such a viewpoint, it is preferable to contain a large amount of an isoparaffin-based hydrocarbon having 10 to 15 carbon atoms. Among these, it is preferable to contain a large amount of isododecane having 12 carbon atoms in terms of a balance between smell, use feeling, and cleansing performance.

Examples of the light liquid isoparaffin include those having trade names of Marukasol R (Maruzen Petrochemical Co., Ltd.), IP Solvent 1620, 2028 (both, Idemitsu Petrochemical Co., Ltd.), Isopar L and Isopar H (both, Exxon Chemical Corporation), and Isosol 300, 400 (both, Nippon Petrochemicals Co., Ltd.), and Marukasol R is preferably used from a viewpoint of containing isododecane at a high purity.

One or more components (B) can be used. In addition, oil having a viscosity of more than 15 mPa·s at 30°C may be used. In this case, the oil composition mixed with the oil of the component (B) preferably has a viscosity of 15 mPa·s or less at 30°C.

The component (B) is contained in an amount of preferably 10 to 40% by weight, more preferably 15 to 30% by weight in the total composition in order that the resulting composition is cleanly washed off without leaving a feeling of residue while maintaining sufficient cleaning power.

Further, the weight ratio of the component (A) to the component (B) is preferably 1:2 to 2:1 ((A) : (B) = 1:2 to 2:1), more preferably 1:1 to 2:1 in order that the resulting composition is cleanly washed off without leaving a feeling of residue while maintaining sufficient cleaning power.

A ratio of the surfactant of the component (A) to the oil of the component (B) is important in order to obtain a solubilized product in which an oil phase becomes a continuous phase as described above. When the content of the surfactant is higher than that of the oil, the oil is captured in the surfactant and an intrinsic makeup raising speed of the oil is lost. On the other hand, when the content of the oil is excessively high with respect to the surfactant, a solubilized state cannot be maintained, resulting in an emulsion. For this reason, it is important that the weight ratio of (A) the surfactant and (B) the oil to be added is as described above.

The water-soluble solvent of the component (C) is not limited as long as it is those typically used for cosmetics, and examples thereof include monohydric alcohols such as ethanol, propanol, isopropanol, butanol and isobutanol; glycols such as ethylene glycol, propylene glycol, diethylene glycol, dipropylene glycol, 1,3-butylene glycol, 1,4-butylene glycol, hexylene glycol and isoprene glycol; glycerols such as glycerol and diglycerol; saccharides such as sorbitol, multitol, maltose, fructose, xylitol, multotriose, threitol, erithritol and glucose; and saccharide derivatives such as methylglucoside and ethylglucoside. Here, a saccharide which is solid at normal temperature forms an aqueous solution with water of the component (D) described later and the aqueous solution can function as a water-soluble solvent.

One or more components (C) can be used and are contained in an amount of preferably 20 to 60% by weight, more preferably 24 to 45% by weight in the total composition because sufficient cleaning power and stability are realized.

The water-soluble solvent of the component (C) further improves stability of the composition of the present invention by containing (C1) a water-soluble solvent having three or more hydroxyl groups in a molecule and (C2) a water-soluble solvent having one or two hydroxyl groups in a molecule.

The water-soluble solvent of the component (C1) has three or more hydroxyl groups in a molecule. Specific examples thereof include glycerols such as glycerol and diglycerol; saccharides such as sorbitol, multitol, maltose, fructose, xylitol, multotriose, threitol, erithritol and glucose; and saccharide derivatives such as methylglucoside and ethylglucoside.
Among these, glycerol, sorbitol and multitol are preferable.

One or more components (C1) can be used and are contained in an amount of preferably 10 to 50% by weight, more preferably 10 to 30% by weight in the total composition because the resulting composition exerts sufficient cleaning power and can be cleanly washed off without leaving a feeling of residue.

The water-soluble solvent of the component (C2) has one or two hydroxyl groups in a molecule. Specific examples thereof include monohydric alcohols such as ethanol, propanol, isopropanol, butanol and isobutanol; and glycols such as ethylene glycol, propylene glycol, diethylene glycol, dipropylene glycol, 1,3-butylene glycol, 1,4-butylene glycol, hexylene glycol and isoprene glycol.
Among these, 1,3-butylene glycol, isoprene glycol, propylene glycol and dipropylene glycol are preferable.

One or more components (C2) can be used, and are contained in an amount of preferably 10 to 50% by weight, more preferably 10 to 30% by weight in the total composition because stability in a wide temperature range and clean wash-off properties without leaving a feeling of residue can be realized.
In addition, the weight ratio of the component (C1) to the component (C2) is 1:3 to 3:1 ((C1): (C2) = 1:3 to 3:1), preferably 1:2 to 2:1 because both sufficient cleaning power and stability in a wide temperature range are realized.

Water of the component (D) is contained in an amount of preferably 5 to 50% by weight, more preferably 10 to 40% by weight in the total composition because the resulting composition can be washed off without an oily feeling while maintaining sufficient cleaning power.
Further, the cleansing composition of the present invention has a total content of the component (C) and the component (D) of 40 to 70% by weight, and preferably 50 to 65% by weight. When the total content is in this range, a feeling of use without an oily feeling and wash-off properties can be obtained, which is preferable.

The cleansing composition of the present invention preferably has an isotropic liquid phase of a bicontinuous structure formed from the components (A), (B), (C) and (D). The isotropic liquid phase of a bicontinuous structure is an optically isotropic transparent or semitransparent solution having a low viscosity in which both water and oil are continuous. Specifically, the isotropic liquid phase of a bicontinuous structure indicates a bicontinuous microemulsion phase (or μE phase), a middle phase (or D phase) or a sponge phase (or L3 phase).

The cleansing composition of the present invention can exhibit a cleaning function, which has never been sufficiently achieved, by preferably having an isotropic liquid phase of a bicontinuous structure. The structure is one in which both water and oil are continuous, and is excellent in cleaning for cosmetics, especially water-resistant mascara and the like. A cosmetic currently used has improved perspiration resistance and sebum resistance, and therefore, it becomes difficult for a conventional cleansing composition to easily wash off the cosmetic. However, a cleansing composition has characteristics that not only affinity for a cosmetic or mascara is excellent, but a wash-off process is greatly facilitated because the composition is very compatible with water even in a wash-off process using water due to an isotropic liquid phase of a bicontinuous structure.

The component (B) can weaken a viscous liquid crystal structure formed from the components (A), (C) and (D) by containing oil having a branched chain, thereby easily forming an isotropic liquid phase of a bicontinuous structure. On the other hand, it is considered that an isotropic liquid phase of a bicontinuous structure that is generally present only in a narrow temperature range is stabilized in a wide temperature range by containing the components (C1) and (C2).

The isotropic liquid phase exhibiting a bicontinuous structure of the cleansing composition according to the present invention can be confirmed by the observation of appearance, observation by an optical polarizing microscope, drawing of a phase diagram, measurement of a self diffusion coefficient by NMR, measurement of electrical conductivity, fluorescent probe method using a fluorescent pigment, or observation by an electronic microscope (such as TEM) in accordance with freeze fracture replica method.

It is possible to distinguish a solution having an isotropic liquid phase exhibiting a bicontinuous structure from the other solution by the observation of appearance, because the former one is in the low-viscosity solution form with a transparent appearance. It is also possible to confirm the isotropic form by arranging two polarizing plates with their polarizing directions perpendicular to each other, and placing, between them, a sample charged in a transparent container. When no light transmission is observed, the solution is confirmed to be isotropic. Through observation by an optical polarizing microscope, the solution can be confirmed to be isotropic if no light transmission is observed at an angle of a polarizing plate set at 90 degree.

Upon confirmation by using a pseudo phase diagram for an aqueous phase (water and water-soluble solvent), an oil phase (oil component) and a surfactant phase, the solution is confirmed to be isotropic if it is in the isotropic liquid form on the phase diagram and is not in a region extending continuously from the apex of the aqueous phase or oil phase. This method is not always applied depending on the substances employed, composition of the aqueous phase or composition of the surfactant phase.

An isotropic liquid phase of a bicontinuous structure can be confirmed only in a narrow temperature range because its structure is very unstable. Accordingly, although its existence as a phase state has been known, its stability in a wide temperature range is problematic and its practical application is thus difficult. However, the present inventors have now found that a cleansing composition having excellent stability and cleaning properties in a wide temperature range can be obtained by using a specific nonionic surfactant, oil, water-soluble solvent and water at a specific ratio in combination.

The cleansing composition of the present invention can optionally further contain components typically used for a cleaning agent, for example, thickeners, bactericides, humectants, moisturizers, colorants, antiseptics, feel improvers, perfumes, anti-inflammatory agents, whitening agents, antiperspirants, ultraviolet absorbers, antioxidants, various extract liquids and the like.

The cleansing composition of the present invention can be obtained by appropriately mixing predetermined components, and can be produced by uniformly mixing all the components irrespective of a mixing sequence after heat-melting a raw material that is solid at normal temperature or dissolving it in the other components.

The cleansing composition of the present invention is a transparent liquid cleansing composition.

"Transparent" refers to a state where turbidity is 500 NTU or less by a turbidimeter (TN-100, manufactured by Eutech Instruments).

Further, "liquid" refers to a state where viscosity is 1000 mPa·s or less at 25°C. In this case, the viscosity is measured by a B-type viscometer (Rotor 2, 30 rpm). The composition of the present invention preferably has a viscosity of 500 mPa·s or less at 25°C.

The following examples further describe and demonstrate embodiments of the present invention. The examples are given solely for the purpose of illustration and are not to be construed as limitations of the present invention.

Examples

Examples 1 to 22 and Comparative Examples 1 to 6

Cleansing compositions with compositions shown in Table 1 to Table 4 were produced and evaluated for makeup removal, an oily feeling during wash-off, stability and transparency. The results are shown in Table 1 to Table 4 altogether.

(Production method)

A cleansing composition was obtained by placing all the components (A) to (D) in a container, and stirring and uniformly mixing all the components.

When a component that is solid at room temperature was present, or when a gel component was formed by mixing at room temperature, the solid component or the gel-forming component was heated at 70 to 75°C while stirring to dissolve it. After thoroughly dissolving the component, the temperature was returned to room temperature to obtain a cleansing composition.

All the cleansing compositions of Examples 1 to 22 were transparent liquid cleansing compositions. Further, these cleansing compositions were confirmed to have an isotropic liquid phase of bicontinuous structure by visual observation and observation by an optical polarizing microscope.

(1) Makeup remover:

0.005 g of Kose Sports Beauty Fasio Powerstay Mascara (Curl long) BK001 (trade name) as an durable mascara (waterproof mascara) was uniformly applied on a glass slide in a circle with a diameter of 1.2 cm and left for 12 hours and then dried. About 25 mg of each of the cleansing compositions was placed on the dried mascara and rubbed with a finger to raise the durable mascara, and then the raised mascara was wiped off with tissue paper to measure the residual amount of the durable mascara. Each of the cleansing compositions was evaluated using the number of times of rubbing until the residual amount of the oil mascara became 0.001 g or less. In this case, the number of times of rubbing when using only 5 mg (20% of 25 mg) of isododecane is used in place of the cleansing composition is 25.

(2) Oily feeling during wash-off:

When about 2 g of each of the cleansing compositions was applied in a forearm portion and washed off with water, an oily feeling during wash-off was evaluated in accordance with the following criteria.

A; There is no oily feeling after wash-off.
B; There is a little oily feeling after wash-off.
C; Oil persistently remains on the skin and cannot be washed off.

(3) Stability:

Each of the cleansing compositions was placed in a glass container with a lid and stored at 50°C and 0°C for one day, and then evaluated for appearance by visual observation in accordance with the following criteria.

A; No change
B; Separation

(4) Transparency:

The turbidity of each of the cleansing compositions was measured by a turbidimeter (TN-100, manufactured by Eutech Instruments) using a measurement sample bottle equipped with the turbidimeter. In this case, a cleansing composition having a turbidity of 500 NTU or less is "transparent".

[Table 1]

| | Component (weight%) | Examples | | | | | | |
|---|---|---|---|---|---|---|---|---|
| | | 1 | 2 | 3 | 4 | 5 | 6 | 7 |
| A | Diglycerol monoisostearate (HLB: 8) [*1] | 7.0 | 7.0 | 7.0 | 7.0 | 5.0 | 7.0 | 5.0 |
| | Polyethylene glycol monolaurate (HLB: 14) [*2] | 14.0 | 14.0 | 14.0 | 14.0 | 11.0 | 14.0 | 11.0 |
| | Alkyl (C8 to C16) glucoside (HLB: 17) [*3] (40% pure aqueous solution) | 10.0 | 10.0 | 10.0 | 10.0 | 7.5 | 10.0 | 7.0 |
| | Polyoxyethylene sorbitol tetraoleate (HLB: 10.5) [*4] | | | | | 3.0 | | 3.0 |
| B | Isododecane (5 mPa·s) | 15.0 | 8.0 | 6.0 | 10.0 | 8.0 | 6.0 | 11.0 |
| | Hydrogenated polyisobutene[*5] (16.5 mPa·s) | | | 2.0 | 5.0 | | | |
| | Isopropyl myristate (10 mPa·s) | | 8.0 | 8.0 | | 8.0 | 8.0 | 11.0 |
| | Decamethylcyclopentasiloxane[*6] (9 mPa·s) | | | | | | 2.0 | |
| | Liquid paraffin[*7] (22.5 mPa·s) | | | | | | | |
| C | Sorbitol | 15.0 | 15.0 | 15.0 | 15.0 | 15.0 | 15.0 | 15.0 |
| | 1,3-butylene glycol | 15.0 | 20.0 | 20.0 | 20.0 | 20.0 | 15.0 | 20.0 |
| | Perfume | 0.2 | 0.2 | 0.2 | 0.2 | 0.2 | 0.2 | 0.2 |
| D | Water | Balance | Balance | Balance | Balance | Balance | Balance | Balance |
| | Total | 100 | 100 | 100 | 100 | 100 | 100 | 100 |
| | Makeup remover (Number of times) | 40 | 41 | 46 | 44 | 44 | 45 | 48 |
| | Oily feeling | A | A | A | A | A | A | A |
| | Stability: 0°C | A | A | A | A | A | A | A |
| | Stability: 50°C | A | A | A | A | A | A | A |

(continued)

| Component (weight%) | Examples | | | | | | |
|---|---|---|---|---|---|---|---|
| | 1 | 2 | 3 | 4 | 5 | 6 | 7 |
| Transparency (Turbidity: NTU) | 27 | 35 | 38 | 39 | 23 | 36 | 40 |

*1: Cosmol 41V (produced by The Nisshin Oillio Group,
*2: Emanon 1112HG (produced by Kao Corporation) Ltd.)
*3: Mydol 10 (produced by Kao Corporation)
*4: Reodol 430 (produced by Kao Corporation)
*5: Parleam Ex (produced by NOF Corporation)
*6: SH-245 (produced By Dow Corning Toray Co., Ltd.)
*7: Hicall K-230 (produced by Kaneda Co., Ltd.)

[Table 2]

| Component (weight%) | | Comparative Examples | | | | | |
|---|---|---|---|---|---|---|---|
| | | 1 | 2 | 3 | 4 | 5 | 6 |
| A | Diglycerol monoisostearate (HLB: 8) *1 | 7.0 | 7.0 | 7.0 | 7.0 | 7.0 | 7.0 |
| | Polyethylene glycol monolaurate (HLB: 14) *2 | 14.0 | 14.0 | 14.0 | 14.0 | 14.0 | 14.0 |
| | Alkyl (C8 to C16) glucoside (HLB: 17) *3 (40% pure aqueous solution) | 10.0 | 10.0 | 10.0 | 10.0 | 10.0 | 10.0 |
| | Polyoxyethylene sorbitol tetraoleate (HLB: 10.5) *4 | | | | | | |
| B | Isododecane (5 mPa·s) | | | 4.0 | | 4.0 | 50.0 |
| | Hydrogenated polyisobutene *5 (16.5 mPa·s) | 15.0 | | | 20.0 | | |
| | Isopropyl myristate (10 mPa·s) | | | | | | |
| | Decamethylcyclopentasiloxane*6 (9 mPa·s) | | | | | | |
| | Liquid paraffin*7 (22.5 mPa·s) | | 15.0 | 12.0 | | | |
| c | Sorbitol | 20.0 | | | | | |
| | 1,3-butylene glycol | | 15.0 | 15.0 | 15.0 | 15.0 | 15.0 |
| | Perfume | 0.2 | 0.2 | 0.2 | 0.2 | 0.2 | 0.2 |
| D | Water | Balance | Balance | Balance | Balance | Balance | Balance |
| | Total | 100 | 100 | 100 | 100 | 100 | 100 |
| | Makeup removal(Number of times) | ≥110 | ≥110 | 92 | 95 | ≥110 | 42 |
| | Oily feeling | B | C | B | B | A | c |
| | Stability: 0°C | B | B | B | B | B | B |
| | Stability: 50°C | B | B | B | B | B | B |
| | Transparency (Turbidity: NTU) | 905 | 985 | 970 | 976 | 951 | 921 |

[Table 3]

| | Component (weight%) | Examples | | | | | | | | |
|---|---|---|---|---|---|---|---|---|---|---|
| | | 8 | 9 | 10 | 11 | 12 | 13 | 14 | 15 | 16 |
| A | Diglycerol monoisostearate (HLB: 8) *1 | 7.0 | 7.0 | 7.0 | 7.0 | 7.0 | 5.0 | 7.0 | 7.0 | 7.0 |
| | Polyethylene glycol monolaurate (HLB: 14) *2 | 14.0 | 14.0 | 14.0 | 14.0 | 14.0 | 11.0 | 14.0 | 14.0 | 8.0 |
| | Alkyl glucoside (HLB: 17) *3 (40% pure aqueous solution) | 10.0 | 10.0 | 10.0 | 10.0 | 10.0 | 7.5 | 10.0 | 10.0 | 10.0 |
| | Polyoxyethylene sorbitol tetraoleate (HLB: 10.5) *4 | | | | | | 3.0 | | | |
| | Glycerol polyoxyethylene coconut oil fatty acid ester (HLB: 13) *8 | | | | | | | | | 7.0 |
| B | Isododecane (5 mPa·s) | 15.0 | 15.0 | 15.0 | 15.0 | 15.0 | 10.0 | 15.0 | 15.0 | 15.0 |
| | Hydrogenated polyisobutene (16.5 mPa·s) *5 | | | | | | 5.0 | | | |
| C1 | Sorbitol | 15.0 | 15.0 | 15.0 | | | 15.0 | 7.5 | 15.0 | 17.5 |
| | Glycerol | | | | 15.0 | | | 7.5 | | |
| | Multitol | | | | | 15.0 | | | | |
| C2 | 1,3-butylene glycol | | | | 20.0 | 20.0 | 20.0 | 20.0 | 10.0 | |
| | Propylene glycol | 20.0 | | | | | | | | |
| | Dipropylene glycol | | 20.0 | | | | | | 10.0 | 25.0 |
| | Isoprene glycol | | | 20.0 | | | | | | |
| | Perfume | 0.2 | 0.2 | 0.2 | 0.2 | 0.2 | 0.2 | 0.2 | 0.2 | 0.2 |
| D | Water | Balance | Balance | Balance | Balance | Balance | Balance | Balance | Balance | Balance |
| | Total | 100 | 100 | 100 | 100 | 100 | 100 | 100 | 100 | 100 |
| | Makeup removal (Number of times) (Number times) | 44 | 46 | 44 | 42 | 39 | 48 | 44 | 47 | 43 |
| | Oily feeling | A | A | A | A | A | A | A | A | A |
| | Stability: 0°C | A | A | A | A | A | A | A | A | A |
| | Stability: 50°C | A | A | A | A | A | A | A | A | A |

(continued)

| Component (weight%) | Examples | | | | | | | | |
|---|---|---|---|---|---|---|---|---|---|
| | 8 | 9 | 10 | 11 | 12 | 13 | 14 | 15 | 16 |
| Transparency (Turbidity: NTU) | 32 | 35 | 45 | 40 | 35 | 28 | 36 | 38 | 49 |
| *8: Unigly MK-207G (produced by NOF Corporation) | | | | | | | | | |

[Table 4]

| Component (weight%) | | Examples | | | | | |
|---|---|---|---|---|---|---|---|
| | | 17 | 18 | 19 | 20 | 21 | 22 |
| A | Diglycerol monoisostearate (HLB: 8) *1 | 3.5 | 5.0 | 7.5 | 5.0 | 5.0 | 5.0 |
| | Polyethylene glycol monolaurate (HLB: 14) *2 | 7.0 | 11.0 | 16.5 | 11.0 | 11.0 | 11.0 |
| | Alkyl glucoside (HLB: 17) *3 (40% pure aqueous solution) | 5.0 | 7.5 | 11.25 | 7.5 | 7.5 | 7.5 |
| | Polyoxyethylene sorbitol tetraoleate (HLB: 10.5) *4 | | 3.0 | 4.5 | 3.0 | 3.0 | 3.0 |
| B | Isododecane (5 mPa·s) | 5.0 | 12.0 | 17.0 | 10.0 | 10.0 | 10.0 |
| | Isopropyl myristate (10 mPa·s) *5 | 5.0 | 12.0 | 17.0 | 10.0 | 10.0 | 10.0 |
| C1 | Sorbitol | 15.0 | 20.0 | 12.0 | 22.0 | 10.0 | 20.0 |
| C2 | 1,3-butylene glycol | 20.0 | 20.0 | 12.0 | 22.0 | 21.0 | 15.0 |
| | Perfume | 0.2 | 0.2 | 0.2 | 0.2 | 0.2 | 0.2 |
| D | Water | Balance | Balance | Balance | Balance | Balance | Balance |
| | Total | 100 | 100 | 100 | 100 | 100 | 100 |
| | Makeup removal (Number of times) | 74 | 32 | 28 | 36 | 62 | 41 |
| | Oily feeling | A | A | A | A | A | A |
| | Stability: 0°C | A | A | A | A | A | A |
| | Stability: 50°C | A | A | A | A | A | A |
| | Transparency (Turbidity: NTU) | 384 | 55 | 43 | 49 | 368 | 68 |

The results of Table 1 to Table 4 showed that there was substantially no oily feeling during use, durable waterproof mascara or the like could be rapidly raised , and it could be washed off without leaving a feeling of residue for all of the cleansing compositions of Examples 1 to 22.

**Claims**

1. A transparent liquid cleansing composition comprising the following components (A), (B), (C) and (D):

(A) 10 to 35% by weight of a nonionic surfactant having an HLB value of 8 or more,
(B) 10 to 40% by weight of oil having a viscosity of 15 mPa·s or less at 30 °C,
(C) 20 to 60% by weight of a water-soluble solvent, and
(D) 5 to 50% by weight of water,

wherein the water-soluble solvent of the component (C) comprises (C1) a water-soluble solvent having three or more hydroxyl groups in a molecule and (C2) a water-soluble solvent having one or two hydroxyl groups in a molecule, and the weight ratio of the component (C1) to the component (C2) is 1:3 to 3:1, and the total content of the components (C) and (D) is 40 to 70% by weight of the cleansing composition.

2. The cleansing composition according to claim 1, wherein the cleansing composition has an isotropic liquid phase of a bicontinuous structure.

3. The cleansing composition according to claim 1 or 2, wherein the weight ratio of the component (A) to the component (B) is 1:2 to 2:1.

4. The cleansing composition according to any one of claims 1 to 3, wherein the component (B) comprises branched hydrocarbon oil.

5. The cleansing composition according to any one of claims 1 to 4, wherein the component (B) comprises 30 to 100% by weight of isoparaffin.

6. The cleansing composition according to any one of claims 1 to 5, wherein the component (B) comprises 30 to 100% by weight of isododecane.

7. The cleansing composition according to any one of claims 1 to 6, wherein the component (C1) is at least one or more selected from the group consisting of glycerol, sorbitol and multitol.

8. The cleansing composition according to any one of claims 1 to 7, wherein the component (C2) is at least one or more selected from the group consisting of 1,3-butylene glycol, isoprene glycol, propylene glycol and dipropylene glycol.

9. Use of a composition comprising the following components (A), (B), (C) and (D):

(A) 10 to 35% by weight of a nonionic surfactant having an HLB value of 8 or more,
(B) 10 to 40% by weight of oil having a viscosity of 15 mPa·s or less at 30 °C,
(C) 20 to 60% by weight of a water-soluble solvent, and
(D) 5 to 50% by weight of water,

wherein the water-soluble solvent of the component (C) comprises (C1) a water-soluble solvent having three or more hydroxyl groups in a molecule and (C2) a water-soluble solvent having one or two hydroxyl groups in a molecule, and the weight ratio of component (C1) to the component (C2) is 1:3 to 3:1, and the total content of the components (C) and (D) is 40 to 70% by weight of the cleansing composition, for removing makeup, especially waterproof mascara.

**Patentansprüche**

1. Transparente flüssige Reinigungszusammensetzung, die die folgenden Komponenten (A), (B), (C) und (D) umfasst:

(A) 10 bis 35 Gew.% eines nicht-ionischen Tensids, das einen HLB-Wert von 8 oder mehr aufweist,
(B) 10 bis 40 Gew.% eines Öls, das eine Viskosität von 15 mPa·s oder weniger bei 30°C aufweist,
(C) 20 bis 60 Gew.% eines wasserlöslichen Lösungsmittels und
(D) 5 bis 50 Gew.% Wasser,

worin das wasserlösliche Lösungsmittel der Komponente (C) ein wasserlösliches Lösungsmittel (C1), das drei oder mehr Hydroxylgruppen im Molekül hat, und ein wasserlösliches Lösungsmittel (C2), das ein oder zwei Hydroxylgruppen im Molekül hat, umfasst, und das Gewichtsverhältnis der Komponente (C1) zur Komponente (C2) 1:3 bis 3:1 ist, und der Gesamtgehalt der Komponenten (C) und (D) 40 bis 70 Gew.% der Reinigungszusammensetzung ist.

2. Reinigungszusammensetzung gemäß Anspruch 1, worin die Reinigungszusammensetzung eine isotrope flüssige Phase mit einer bikontinuierlichen Struktur aufweist.

**3.** Reinigungszusammensetzung gemäß Anspruch 1 oder 2, worin das Gewichtsverhältnis der Komponente (A) zur Komponente (B) 1:2 bis 2:1 ist.

**4.** Reinigungszusammensetzung gemäß irgendeinem der Ansprüche 1 bis 3, worin die Komponente (B) verzweigtes Kohlenwasserstofföl umfasst.

**5.** Reinigungszusammensetzung gemäß irgendeinem der Ansprüche 1 bis 4, worin die Komponente (B) 30 bis 100 Gew.% Isoparaffin umfasst.

**6.** Reinigungszusammensetzung gemäß irgendeinem der Ansprüche 1 bis 5, worin die Komponente (B) 30 bis 100 Gew.% Isododekan umfasst.

**7.** Reinigungszusammensetzung gemäß irgendeinem der Ansprüche 1 bis 6, worin die Komponente (C1) mindestens ein oder mehrere ausgewählt aus der Gruppe bestehend aus Glycerin, Sorbitol und Multitol ist.

**8.** Reinigungszusammensetzung gemäß irgendeinem der Ansprüche 1 bis 7, worin die Komponente (C2) mindestens ein oder mehrere ausgewählt aus der Gruppe bestehend aus 1,3-Butylenglykol, Isoprenglykol, Propylenglykol und Dipropylenglykol ist.

**9.** Verwendung einer Zusammensetzung, die die folgenden Komponenten (A), (B), (C) und (D) umfasst:

(A) 10 bis 35 Gew.% eines nicht-ionischen Tensids, das einen HLB-Wert von 8 oder mehr aufweist,
(B) 10 bis 40 Gew.% eines Öls, das eine Viskosität von 15 mPa·s oder weniger bei 30°C aufweist,
(C) 20 bis 60 Gew.% eines wasserlöslichen Lösungsmittels und
(D) 5 bis 50 Gew.% Wasser,

worin das wasserlösliche Lösungsmittel der Komponente (C) ein wasserlösliches Lösungsmittel (C1), das drei oder mehr Hydroxylgruppen im Molekül hat, und ein wasserlösliches Lösungsmittel (C2), das ein oder zwei Hydroxylgruppen im Molekül hat umfasst, und das Gewichtsverhältnis der Komponente (C1) zur Komponente (C2) 1:3 bis 3:1 ist, und
der Gesamtgehalt der Komponenten (C) und (D) 40 bis 70 Gew.% der Reinigungszusammensetzung ist,
zum Entfernen von Make-up, insbesondere wasserfestem Mascara.

## Revendications

**1.** Composition de liquide transparent de nettoyage comprenant les composants suivants (A), (B), (C) et (D) :

(A) de 10 à 35% en poids d'un tensio-actif non ionique ayant une valeur HLB supérieure ou égale à 8,
(B) de 10 à 40% en poids d'une huile ayant une viscosité inférieure ou égale à 15 mPa·s à 30°C,
(C) de 20 à 60% en poids d'un solvant hydrosoluble, et
(D) de 5 à 50% en poids d'eau,

dans laquelle le solvant hydrosoluble du composant (C) comprend un solvant hydrosoluble (C1) ayant trois groupes hydroxyle ou plus dans une molécule et un solvant hydrosoluble (C2) ayant un ou deux groupe(s) hydroxyle dans une molécule, et le rapport massique du composant (C1) sur le composant (C2) varie de 1:3 à 3:1, et
la teneur totale en composants (C) et (D) est de 40 à 70% en poids de la composition de nettoyage.

**2.** Composition de nettoyage selon la revendication 1,
dans laquelle la composition de nettoyage a une phase liquide isotrope d'une structure bicontinue.

**3.** Composition de nettoyage selon la revendication 1 ou 2,
dans laquelle le rapport massique du composant (A) sur le composant (B) varie de 1:2 à 2:1.

**4.** Composition de nettoyage selon l'une quelconque des revendications 1 à 3, dans laquelle le composant (B) comprend une huile d'hydrocarbure ramifié.

**5.** Composition de nettoyage selon l'une quelconque des revendications 1 à 4, dans laquelle le composant (B) comprend 30 à 100% en poids d'isoparaffine.

6. Composition de nettoyage selon l'une quelconque des revendications 1 à 5, dans laquelle le composant (B) comprend 30 à 100% en poids d'isododécane.

7. Composition de nettoyage selon l'une quelconque des revendications 1 à 6, dans laquelle le composant (C1) est au moins un ou plusieurs élément(s) choisi(s) dans le groupe constitué de glycérol, sorbitol et de multitol.

8. Composition de nettoyage selon l'une quelconque des revendications 1 à 7, dans laquelle le composant (C2) est au moins un ou plusieurs élément(s) choisi(s) dans le groupe constitué du 1,3-butylène glycol, de l'isoprène glycol, du propylène glycol et du dipropylène glycol.

9. Utilisation d'une composition comprenant les composants suivants (A), (B), (C) et (D) :

(A) de 10 à 35% en poids d'un tensio-actif non ionique ayant une valeur HLB supérieure ou égale à 8,
(B) de 10 à 40% en poids d'une huile ayant une viscosité inférieure ou égale à 15 mPa·s à 30°C,
(C) de 20 à 60% en poids d'un solvant hydrosoluble, et
(D) de 5 à 50% en poids d'eau,

où le solvant hydrosoluble du composant (C) comprend un solvant hydrosoluble (C1) ayant trois groupes hydroxyle ou plus dans une molécule et un solvant hydrosoluble (C2) ayant un ou deux groupe(s) hydroxyle dans une molécule, et le rapport massique du composant (C1) sur le composant (C2) varie de 1:3 à 3:1, et la teneur totale en composants (C) et (D) est de 40 à 70% en poids de la composition de nettoyage, pour enlever le maquillage particulièrement un mascara résistant à l'eau.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4005213 A
- JP 2000256124 A
- JP 2000256132 A
- JP 2004339212 A
- JP 2004217640 A
- EP 1053740 A
- JP 04091018 B
- JP 2006117643 A
- JP 2004359568 A
- JP 2002114623 A
- JP 6016524 A
- JP 6016523 A